# EUROPEAN PATENT SPECIFICATION

(11) **EP 2 217 930 B1**
(45) Date of publication and mention of the grant of the patent: **06.03.2013**
(21) Application number: 08843255.4
(22) Date of filing: 24.10.2008
(51) Int. Cl.: G01N 33/68

(54) **MALDI MS-BASED HIGH-THROUGHPUT SCREENING METHOD FOR SUBSTANCES INHIBITING AGGREGATION OF ALZHEIMER'S AMYLOID BETA PEPTIDES**
SCREENING-VERFAHREN AUF BASIS VON MALDI-MS MIT HOHEM DURCHSATZ FÜR DIE AGGREGATION VON ALZHEIMER-AMYLOID-BETA-PEPTIDEN HEMMENDE SUBSTANZEN
PROCÉDÉ DE CRIBLAGE À GRANDE CADENCE À BASE DE MALDI MS POUR DES SUBSTANCES QUI INHIBENT L'AGRÉGATION DES PEPTIDES BÊTA-AMYLOÏDES DE LA MALADIE D'ALZHEIMER

(30) Priority: 24.10.2007 US 982303 P
(43) Date of publication of application: 18.08.2010
(73) Proprietor: Tallinn University of Technology, 19086 Tallinn (EE)
(72) Inventor: PALUMAA, Peep, 10147 Tallinn (EE); TUULING, Marina, 13419 Tallinn (EE); BLAZEVITZ, Olga, 13911 Tallinn (EE); KAZANTZEVA, Jekaterina, 13811 Tallinn (EE); SABAROVA, Irina, 13523 Tallinn (EE); ZOVO, Kairit, 10118 Tallinn (EE)
(74) Representative: Koppel, Mart Enn
(86) International application number: PCT/EE2008/000024
(87) International publication number: WO 2009/052837

(56) References cited:
- CHENG XUN ET AL: "Mass spectrometry-based screening for inhibitors of beta-amyloid protein aggregation" ANALYTICAL CHEMISTRY, AMERICAN CHEMICAL SOCIETY. COLUMBUS, US, vol. 77, no. 21, 1 November 2005 (2005-11-01), pages 7012-7015, XP008101567 ISSN: 0003-2700 [retrieved on 2005-10-04] cited in the application
- FARMER T B ET AL: "Mass Discrimination in Matrix-assited Laser Desorption Ionization Time-of-Flight Mass Spectrometry: a Study Using Cross-linked Oligomeric Complexes" JOURNAL OF MASS SPECTROMETRY, WILEY, CHICHESTER, GB, vol. 30, 1 January 1995 (1995-01-01), pages 1245-1254, XP002430802 ISSN: 1076-5174
- BOLBACH GERARD: "MATRIX-ASSISTED LASER DESORPTION/IONIZATION ANALYSIS OF NON-COVALENT COMPLEXES: FUNDAMENTALS AND APPLICATIONS" CURRENT PHARMACEUTICAL DESIGN, BENTHAM SCIENCE PUBLISHERS, SCHIPHOL, NL, vol. 11, no. 20, 1 January 2005 (2005-01-01), pages 2535-2557, XP008077825 ISSN: 1381-6128
- ROHNER T C ET AL: "MALDI mass spectrometric imaging of biological tissue sections", MECHANISMS OF AGEING AND DEVELOPMENT, ELSEVIER SEQUOIA, LAUSANNE, CH, vol. 126, no. 1, 1 January 2005 (2005-01-01), pages 177-185, XP004689563, ISSN: 0047-6374, DOI: DOI:10.1016/J.MAD.2004.09.032

## Description

### TECHNICAL FIELD

The invention relates to the field of pharmaceutical research tools, namely to methods for screening substances that inhibit aggregation of Aβ peptides. Such substances have a potential for treatment or prevention of Alzheimer's disease.

### BACKGROUND ART

Alzheimer's disease (AD) is age-dependent neurodegenerative disorder, which affects nearly 10% of people over the age of 65. Currently there is no cure or effective treatment available for AD patients (Blennow et al., 2006). As advances in nutrition and medicine lead to increased average lifespan of human population, the number of people afflicted by AD is expected to increase dramatically. Clinical signs of AD include progressive cognitive deterioration, together with declining activities of daily living and by neuropsychiatric symptoms or behavioral changes (Blennow et al., 2006). AD is long-lasting disease, which is connected with high and increasing care and medication costs. Given these trends, there is a tremendous need to develop effective therapeutics that block or reverse AD.

A number of current drugs, which are in clinical use for the treatment of AD, target the symptoms of the disease, rather than its underlying molecular cause and new drugs that disrupt the underlying molecular etiology of AD are in development (Blennow et al., 2006).

Description of AD pathology by Alois Alzheimer in 1906 as well as definitive diagnosis of AD still now includes presence of proteinaceous aggregates in the brain (Blennow et al., 2006; Hardy, 2006b). Major extracellular aggregates characteristic for AD - amyloid plaques are mainly composed of so called amyloid beta (Aβ) peptides (Roher et al., 1993), and numerous genetic and biochemical studies suggest that aggregation of these Aβ peptides, thereby forming amyloid fibrils and plaques, plays a causative role in the development of AD (Hardy, 2006a). Therefore, compounds that inhibit production and/or aggregation of Aβ peptides are attractive candidates as therapeutics for the prevention and/or treatment of AD (LeVine, 2004; Rochet, 2007).

Aβ peptides are produced in vivo by consequtive proteolytic cleavage of the amyloid precursor protein (APP) by β- and γ- secretases (Blennow et al., 2006). γ-secretase can cleave APP within its transmembrane region at two major sites by releasing Aβ peptides composed of 40 and 42 residues. Aβ40 is produced in greater abundance; however, Aβ42 aggregates more readily and comprises the major component of amyloid plaques (Gravina et al., 1995; Roher et al., 1993). It is known that mutations in the amyloid precursor protein (APP) and γ-secretase genes (PS-1 and PS-2), associated with familial forms of AD [14] result in an increased Aβ production or their amyloidogenicity (Haass and Selkoe, 2007; Selkoe, 2005; Tanzi and Bertram, 2005), which supports the importance of amyloid pathway in AD.

The whole amyloid pathway in its complexity is an attractive target for therapeutic intervention, whereas affecting of different steps connected with production and/or aggregation of Aβ peptides might equally lead to the desired effect - prevention of amyloid formation. Major therapeutic strategies of AD are connected with (i) affecting of proteolytic cleavage of APP into Aβ peptides, (ii) clearance of Aβ peptides from the system, and (iii) inhibition of Aβ aggregation into oligomers and insoluble amyloid (Blennow et al., 2006).

Numerous studies have targeted the first two upstream processes by designing selective secretase inhibitors and Aβ directed immunotherapy (McGeer and McGeer, 2005), however, so far these attempts have not produced effective pharmaceuticals. At the same time these studies have also revealed problems connected with application of secretase inhibitors (Beher and Graham, 2005) as well as with Aβ immunotherapy (Morgan, 2006) and latter approach has gone through a serious drawback connected with passive immunization of AD patients with Aβ (Gelinas et al., 2004).

Many efforts have also been focused on the Aβ aggregation step itself with the aim to find effective aggregation inhibitors (LeVine, 2004; Ritchie et al., 2004; Rochet, 2007). The discovery of compounds that block Aβ aggregation has been complicated by two major hindrances. First, structure-based rational drug design is largely precluded as peptides are conformationally labile and not much is known from the composition and structure of the Aβ species prone to aggregation. Second, large-scale screening of combinatorial libraries has been hindered by the unavailability of a cost-effective high-throughput screening methods for inhibitors of the early steps of Aβ aggregation.

Although several methods to monitor Aβ aggregation are available, and many of them have been used for screen for inhibitors of Abeta aggregation (Blanchard et al., 2004; Esler et al., 1997; Necula et al., 2007), these methods are hampered by several shortcomings. Methods, connected with the detection of Aβ fibrils by turbidimetry (2005) or by fluorescent dyes (Necula et al., 2007) typically require relatively large quantities of synthetic Aβ peptide, which is too expensive to use in screens with large libraries of compounds. Moreover, methods based on binding of various fluorogenic dyes to Aβ fibrils (Necula et al., 2007) can generate false positive results. There are also various cellular methods for probing of Aβ aggregation elabotared (2004; Hong et al., 2007; Kim et al., 2006), however, environment in cellular systems is very complex and poorly defined and tested compounds shall pass cellular membranes or bacterial cell walls before affecting the aggregation of intracellularly expressed Aβ peptides or their fusion proteins (Kim et al., 2006).

Modern mass spectrometric techniques have also been applied in studies of amyloid peptides. Matrix assisted laser desorption ionization mass spectrometry (MALDI MS) has been successfully used for imaging of Aβ in brain tissue sections (Rohner et al., 2005), however, latter method has not been used for screening the inhibitors of Aβ aggregation. Liquid chromatography (LC) coupled with electrospray ionization mass spectrometry (ESI MS) has been applied for screening of inhibitors of Aβ40 amyloid formation (Cheng and van Breemen, 2005), however, LC-MS is generally connected with difficulties in automation and low speed of analyses.

Thus, there is a need for a new high throughput method for screening of inhibitors of Aβ peptide aggregation.

### DISCLOSURE OF THE INVENTION

One goal of the present invention is using a Matrix-Assisted Laser Desorption Ionization (MALDI) Time-Of-Flight (TOF) mass spectrometry (MS) as a screening test for fast and sensitive detection of monomeric synthetic Aβ peptides (including Aβ40 and Aβ42 peptides, and their mixtures) during their aggregation.

A second goal of the present invention is using an internal MALDI MS standard for quantification of content of Aβ peptide in the sample. Such a standard is based on using a spectrum of a known compound, such as human insulin (preferably B chain human insulin), as a reference. The invention in its broadest sense is defined by independent claims 1 and 8: 1. A method for screening a plurality of test compounds for inhibitors of amyloid beta (Aβ) peptide aggregation with Matrix-Assisted Laser Desorption Ionization Mass Spectroscopy (MALDI MS), comprising: providing a matrix for MALDI MS; providing an internal MS standard for MALDI MS, the internal MS standard comprising a solution of a known component with a suitable molecule size for quantification of MALDI MS process; providing a monomer amyloid beta (Aβ) peptide sample in a first well of a well plate providing a plurality of solutions in a plurality of remaining wells of said well plate, each of said solutions comprising one test compound to be screened and said monomer Aβ peptide sample; incubating all of the samples for a predetermined time; mixing of said samples from said well plate with said matrixand said internal MS standard; registering a MALDI TOF MS spectrum for said Aβ peptide and said internal MS standard; determiningaMS peak intensity from said MS spectrum; calculating a ratio of peak intensities for said Aβ peptide and said internal MS standard; and determining each test compound as potential therapeutic agent if the ratio of peak intensities for said monomer Aβ peptide and said internal MS standard in the presence of test compound is higher than said ratio of peak intensities for said monomer Aβ peptide and said internal MS standardalone. 8. method for screening a test component for Alzheimer's drug candidates, the method comprising the steps of: preparing a first sample,comprisinga monomer Aβ peptide; preparing a second sample, comprising said monomer Aβ peptide and a test component; incubating said prepared samples for a predetermined time; mixing said incubated samples with a MALDI MS matrix, to form first mixture and second mixture, wherein the MALDI-MS matrix containing a standard component; registering a first spectrum of said first mixture by a MALDI MS, said first spectrum containing a first peak intensity for said peptide component and a second peak intensity for said standard component; calculating a first ratio of said first peak intensity to said second peak intensity; registering a second spectrum of said second mixture by the MALDI MS, said spectrum containing a third peak intensity for said peptide component and a fourth peak intensity for said standard component; calculating a second ratio of said third peak intensity to said fourth peak intensity; and determining said test component as a potential therapeutic agent for treating Alzheimer if said second ratio is higher than said first ratio. Preferred embodiments are defined in dependent claims 2-7 and 9-13. The main advantages of the invention are the possibility to work with a well defined system containing only native Aβ peptides without interfering substances and using a MALDI spotting robot for enabling high throughput screening.

The present invention provides a sensitive and fast *in vitro* screening test for inhibitors of Alzheimer's peptide aggregation based on quantitative MALDI time-of-flight (TOF) mass spectrometric detection of monomeric Aβ42. The major advantages of our assay over other current screening methods are connected with the possibility to work with well-defined systems as well as with high sensitivity and speed. As compared to the very common fluorimetric Thioflavin T (ThT) assay at least 100 times less of Aβ42 is used for measurements. Elaborated automatic MALDI spotting from 96-well plates to 100-well MALDI plates combined with automated MS measurements enables testing of 96 samples within 2.5 hours, i.e., it is a high-throughput method compared to alternatives. The MALDI-TOF MS based screening test was successfully evaluated with ThT assay as well as by transmission electron microscopy (TEM). 35 selected substances were tested for their ability to inhibit Aβ42 aggregation and it was found that from the organic substances tested, only hemin inhibits aggregation of Aβ42. Biometals like zinc and copper also inhibited aggregation of Aβ42 and their effect on the kinetics of aggregation was studied by MALDI-TOF MS in more detail. Kinetic study revealed a concentration dependent inhibitory effect of both tested biometals on aggregation of Aβ42. Elaborated *in vitro* screening test is well applicable for testing the modulators of Aβ42 aggregation as well as amyloid degrading substances, which might serve as potential drugs for AD.

The features and advantages described herein are not all-inclusive and, in particular, many additional features and advantages will be apparent to one of ordinary skill in the art in view of the drawings, specification, and claims. Moreover, it should be noted that the language used in the specification has been principally selected for readability and instructional purposed, and not to limit the scope of the inventive subject matter.

### BRIEF DESCRIPTION OF THE DRAWINGS

Fig. 1 depicts MALDI-TOF MS calibration curves for insulin B chain with different matrices: 1) open square - α-cyano; 2) open circle - DHB; 3) open triangle - HABA.
Fig. 2 depicts MALDI-TOF MS calibration curve for insulin B chain. The concentration corresponds to the initial concentration of the peptide; concentration of B chain in the MALDI MS spot was two times lower.
Fig. 3 depicts MALDI-TOF MS calibration curve for insulin B chain in the linear dynamic range. The concentration corresponds to the initial concentration of the peptide; concentration of B chain in the spot was two times lower.
Fig. 4 depicts MALDI-TOF MS calibration curve of the ratio of Aβ42 and B chain. The concentration shown on the graph represents the initial concentration of the Aβ42 peptide; concentration in the spot was four times lower.
Fig. 5 depicts the aggregation curve of Aβ42 peptide obtained with automatic MALDI-TOF MS method. Line - fitted first order kinetic curve.
Fig. 6 depicts the aggregation curve of Aβ42 peptide obtained with automatic MALDI-TOF MS method. Line - fitted first order kinetic curve.
Fig. 7 depicts The aggregation curve of Aβ40 peptide obtained with automatic MALDI-TOF MS method.
Fig. 8 depicts the aggregation curve of Aβ42 peptide monitored by Thioflavin T assay. Line - fitted first order kinetic curve.
Fig. 9 depicts the effect of vitamins and other selected compounds on aggregation of Aβ42 obtained by MALDI-TOF MS. Control - intensity of Aβ42 monomer peak after 0 or 2 h incubation without additional substances; other columns - intensity of Aβ42 monomer peak after 2 h incubation with 10 or 100 µM concentration of tested substance.
Fig. 10 depicts the effect of antioxidants and other selected compounds on Aβ42 aggregation obtained by MALDI-TOF MS. Control - intensity of Aβ42 monomer peak after 0 or 2 h incubation without additional substances; other columns - intensity of Aβ42 monomer peak after 2 h incubation with 10 µM tested substance.
Fig. 11 depicts the effects of metal ions on Aβ42 aggregation obtained by MALDI-TOF MS. Control - intensity of Aβ42 monomer peak after 0 or 2 h incubation without additional substances; other columns - intensity of Aβ42 monomer peak after 2 h incubation with various concentrations of tested metal ion.

### MODES FOR CARRYING OUT THE INVENTION

The different steps of the method according to the invention are described in detail below.

### Materials

Both 40 and 42 amino acid amyloid beta peptides were purchased from rPeptide (Bogart, USA). Insulin was purchased from Novo Nordisk A/S (Bagsværd, Denmark). Matrices α-cyano (α-cyano-4-hydroxycinnamic acid), Sinapinic acid (3, 5-Dimethoxy-4-hydroxy cinnamic acid) and DHB (2, 5-dihydroxybenzoic acid) were from Fluka (Switzerland) and HABA (2-(-4-hydroxyphenylazo)-benzoic acid) from Sigma (USA). Reagents were purchased as follows: TFA (trifluoroacetic acid) from Riedel de-Haën (Seelze, Germany), acetonitrile from Rathburn (Walkerburn, UK), HEPES (4-(2-hydroxyethyl)-1-piperazineethanesulfonic acid) from USB Corporation (Cleveland, USA) and sodium chloride from Scharlau (Barcelona, Spain). 96-well plates were Greiner Polypropylene Multiwell clear V-bottom plates resistant to chemicals and were purchased from Sigma. Tested compounds were purchased as follows: Zn(CH₃COO)₂ from Scharlau, Mg(CH₃COO)₂ from Merck (Darmstadt, Germany), bis-ANS (4,4'-dianilino-1,1'-binaphtyl-5,5'-disulfonic acid, dipotassium salt) from Invitrogen (Eugene, USA) and all other compounds of analytical grade or higher were from Sigma.

### Methods

### Selecting the MALDI MS matrix

Testing of different matrices and their composition is necessary for selecting of the most suitable matrix yielding the highest sensitivity and reproducibility in detection of biomolecules by MALDI MS. We have considered three possible matrix candidates that are suitable for peptides:
1) α -cyano-4-hydroxycinnamic acid (α-cyano) - suitable for peptides with a molecular mass lower than 10 kDa. A fresh solution has to be prepared weekly.
2) 2, 5-dihydroxybenzoic acid (DHB) - suitable for small molecules and peptides. A fresh solution has to be prepared weekly.
3) 2-(-4-hydroxyphenylazo)-benzoic acid (HABA) - suitable for mixtures of peptides. A fresh solution has to be prepared weekly.

Matrix solutions were prepared as follows: 1) α-cyano 10 mg/ml in 50% AcN 0.2% TFA; 2) DHB 10 mg/ml in 50% AcN and 3) HABA 1.3 mg/ml in 50% AcN. After dissolving, the solutions were centrifuged for 5 min at 7000 rpm and then supernatant was collected.

To test the matrices, a peptide standard (produced as described in the next chapter) with varying concentrations (2.5, 5, 10, 25 and 50 µM) in 20% ethanol was used. The standard was mixed with matrix solution at a ratio of 1:3, and spots were air-dried. MALDI spectra were registered with a Voyager DE™ STR BioSpectrometry™ Workstation (Applied Biosystems, USA) in reflector mode, using following instrument parameters:
Accelerating Voltage: 25000 V
Grid Voltage: 65%
Delay Time: 500 ns
Shots/Spectrum: α-cyano - 100, DHB - 50, HABA - 30
Mass Range: 1500 -10000 Da
Low Mass Gate: 600 Da
Laser Intensity: 2050 V
Number of spectra accumulated -10

The same MALDI-TOF MS experiments were conducted in the same conditions using varying concentrations of Aβ42 peptide (0.5, 1, 2.5, 5 and 10 µM) dissolved in cold 0.02% ammonium hydroxide. The spectra were processed with Data Explorer™ Software (Version 4; Applied Biosystems, USA).

### Production of the insulin B chain

The initial solution used for production of the insulin B chain was 3.5 mg/ml (0.6 mM) synthetic human insulin (Novo Nordisk A/S, Denmark). The initial solution was diluted 3 times in 20mM HEPES 100mM NaCl pH 7.3 buffer to the final concentration of 0.2 mM. Insulin was reduced with 5mM dithiothreitol (DTT) - incubation lasted for 2 hours and the reduction process was monitored with MALDI-TOF MS. The products of reduction reaction were alkylated with iodoacetamide (final concentration 25 mM) in the dark for approximately 1 hour. The products were separated by RP-HPLC in conditions described below:
Column:
   - Agilent Eclipse XDB-C18 (150 x 4.6)
   - Column Volume: 2.493 ml
   - Max Pressure: 6.15 MPa (61,5 Bar)
   - Column Flow: 1 ml/min
Method parameters:
   - Buffer A: 0.1 % TFA
   - Buffer B: 95% AcN 0.1% TFA
   - Flow rate: 1 ml/min
   - Gradient: 30% - 45% of buffer B over 5 column volumes
0.25 ml fractions were collected on a 96 well plate and analyzed by MALDI-TOF MS. Fractions containing B chain of insulin were pooled, freezed to -80°C and lyophilized.

### MALDI-TOF MS calibration curves

To test the quantitative nature of the used MALDI-TOF MS method, a graph showing the relationship between the concentration of insulin B chain and corresponding MS peak intensity was generated. α-cyano matrix with a concentration of 10mg/ml (50% AcN, 0.2% TFA) was used. The initial B chain stock solution and dilutions (20, 17.5, 15, 12.5, 10, 7.5, 5, 2.5, 1, 0.5, 0.25 µM) were made in 20% ethanol. Spots on MALDI plates were generated automatically with a MALDI spotter as described below. MS measurements were performed in reflector mode described above.

Additionally a calibration curve presenting the intensity ratio of Aβ42 and B chain in the spot was generated. The concentration of Aβ42 was varied, and the concentration of the B chain was kept constant (0.25 µM). The dilutions of Aβ42 were made in 0.02% ammonium hydroxide (0.0625, 0.125, 0.25, 0.5, 1, 2, 3, 4, 5, 6, 7, 8, 9 and 10 µM). B chain was added to the matrix solution (α-cyano, 10 mg/ml), which was prepared in 60% AcN 0.3% TFA. Spots on MALDI plates were generated automatically with a MALDI spotter as described below. Mass spectrometric measurements were performed in linear mode using following instrument parameters:
Accelerating Voltage: 25000 V
Grid Voltage: 91%
Delay Time: 650 ns
Shots/Spectrum: 50
Mass Range: 1500 - 10000 Da
Low Mass Gate: 1000 Da
Laser Intensity: 2194 V
Number of spectra accumulated -10

The spectra were processed with Data Explorer™ Software (Version 4; Applied Biosystems, USA).

### Solubilization of Aβ42

For every aggregation experiment Aβ42 solutions were prepared following the same procedure. The lyophilized peptide was taken out from the -80°C fridge, and incubated at room temperature until thawing. Cold 0.02% ammonium hydroxide was added to the peptide to get a final concentration of 10 µM. The solution was mixed on the vortex for a minute and put on ice. Before the beginning of the experiment the solution was mixed again for a shorter time. To start the experiment, the peptide stock solution was diluted in a ratio of 1:1 into the buffer, which contained various concentrations of a tested compound. All probes contained 5 µM Aβ42 in 20mM HEPES 0.1 M NaCl pH 7.5. Aβ42 solutions were always prepared shortly before the beginning of experiment.

### Description of manual MALDI MS method

First, α-cyano matrix was diluted in 50% AcN 0.2% TFA to a final concentration of 10 mg/ml. The solution was mixed on a vortex, centrifuged for 5 min at 6000 rpm and then the supernatant was removed. The eppendorf with matrix solution was covered with folium to prevent degradation by light. A 2.5 µM B chain standard solution in 20% ethanol was prepared. In manual mode the spots on MALDI plates were generated as follows:
1. 3 µl of matrix was pipetted into one well of the mixing plate.
2. 1 µlof peptide standard was pipetted into another well.
3. 1 µl of Aβ sample was pipetted into the well containing standard and mixed.
4. 1 µl of the probe/standard solution was pipetted into the 3 µl of matrix solution.
5. The solution was thoroughly mixed and 2x1µl was transferred to the MALDI 100-well gold plate to generate two parallel spots.

The spots were air-dried and then spectra were registered with Voyager DE™ STR BioSpectrometry™ Workstation using the parameters described in paragraphs [0040]-[0047]. The spectra were processed with Data Explorer™ Software (Version 4; Applied Biosystems, USA).

### Description of automated MALDI MS method

First, 5 µM B chain solution in 60% AcN 0.3% TFA was prepared and added to the α-cyano matrix solution to generate a 10 mg/ml α-cyano, 0.25 µM B chain solution in 60% AcN 0.3% TFA. The solution was mixed on a vortex for a minute and then incubated at room temperature for 10 minutes. Solution was mixed again, centrifuged for 3 min at 6000 rpm and the supernatant was transferred into a clean vial. Shortly before the beginning of aggregation experiments, Aβ42 solutions were prepared and transferred to sequential wells of a 96-well plate.

Spots on MALDI plates were generated automatically by an Ettan Spotter (Amersham Biosciences, Sweden). Different spotting procedures were tested and best results were obtained using the following method: 0.5 µl sample + 1.5 µl matrix containing the internal calibrant - mixing 3 times with a volume of 1 µl.

The spots were air-dried and spectra were collected automatically with Voyager DE™ STR BioSpectrometry™Workstation (Applied Biosystems, USA) mass spectrometer. Automation was controlled by a program called Sequence Control (Applied Biosystems, USA) and instrument parameters described in paragraphs [0065]-[0072] were used. The spectra were processed with Data Explorer™ Software (Version 4; Applied Biosystems, USA).

### Description of Thioflavin T fluorescence assay

Aβ aggregation was followed also by the common Thioflavin T fluorescence assay. Aβ42 aggregation experiments were conducted in 20 mM HEPES 0.1 M NaCl pH 7.3 containing 3.3 µM ThT and 5 µM Aβ42. The fluorescence measurements were performed on a Perkin-Elmer LS-45 Luminescence Spectrometer (USA) at λex = 440 nm and λem = 480 nm.

The concentration dependent effects of Zn²⁺ and Cu²⁺on Aβ42 aggregation were determined with 3.3 µM ThT in 50 mM glycine buffer pH 9.0. The Aβ42 probes, taken after the end of the MALDI-TOF MS experiment, were added into ThT containing buffer to a final concentration of 1.7 µM. The fluorescence measurements were performed as described above.

### RESULTS

### Selecting the matrix

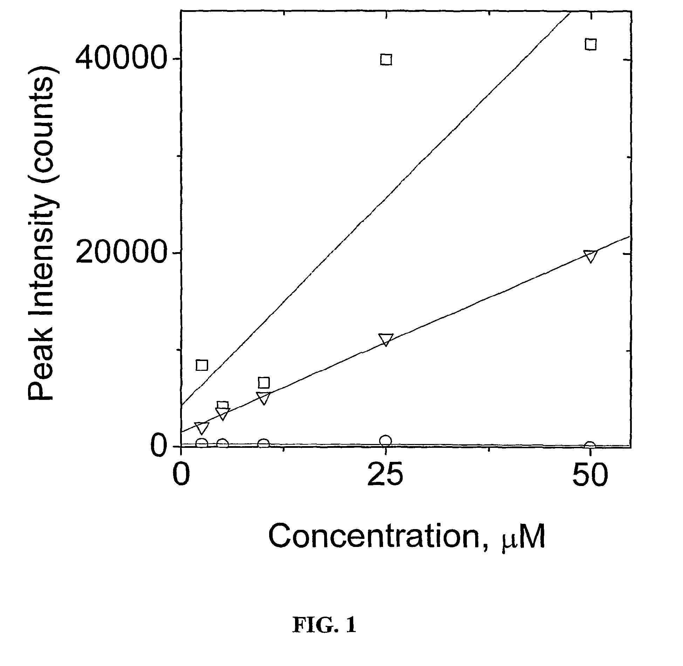

α-cyano and HABA matrices both produced acceptable calibration curves for B chain, whereas application of DHB matrix resulted in very low peak intensities (Fig. 1.). Therefore, we decided to test first two matrices further with Aβ42 peptide using the same experimental conditions. With Aβ42 peptide HABA failed to produce acceptable spectra, but results with α-cyano were as good as with the B chain (data not shown). Based on these results, we chose α-cyano matrix for our further experiments.

### Production of insulin B chain

Insulin itself was not a good internal standard for MALDI-TOF MS experiments, because its solution was not stable. Therefore we decided to separate the two chains (A and B) of insulin, linked by two disulphide bonds and use only one chain as a standard. The disulphide bonds were reduced with 5 mM DTT and the process was monitored by MALDI-TOF MS. By adding 5 mM DTT, B chain emerges within few minutes and the process is completed within 2 h. Moreover, B chain ionizes better than insulin itself and the A chain, which justifies its application as an internal standard in MALDI-TOF MS experiments.

For stabilization, the peptides were alkylated with iodoacetamide to prevent the recovery of disulphide bonds. The separation of two chains of insulin was performed by RP-HPLC. The peaks indicated in the chromatogram were analyzed by MALDI-TOF MS and molecular mass (3547 Da) obtained from the major peak corresponds to the theoretical molecular weight of carboxamidomethylated insulin B chain and according to MS analysis the product was homogeneous. The fractions containing B-chain were freezed, lyophilized and the obtained peptide was used in our MALDI-TOF MS experiments as an internal standard.

### Calibration curves

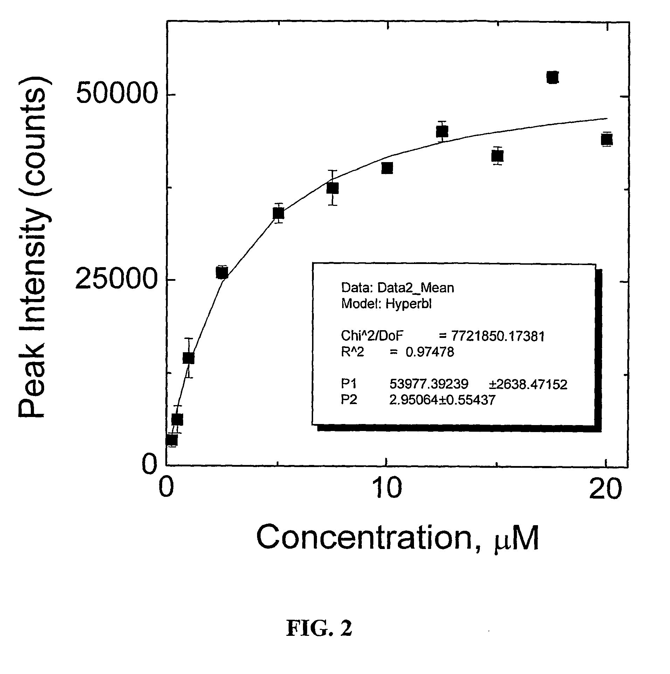

MALDI-TOF MS could be applied for quantitative measurements of peptides by using internal standards. We elaborated MALDI-TOF MS for quantitative measurements of the Alzheimer's amyloid peptides by using of insulin B chain as internal standard. To validate the applicability of MALDI-TOF MS method for quantitative measurements of peptides, we initially generated a calibration curve for insulin B chain (Fig.2.).

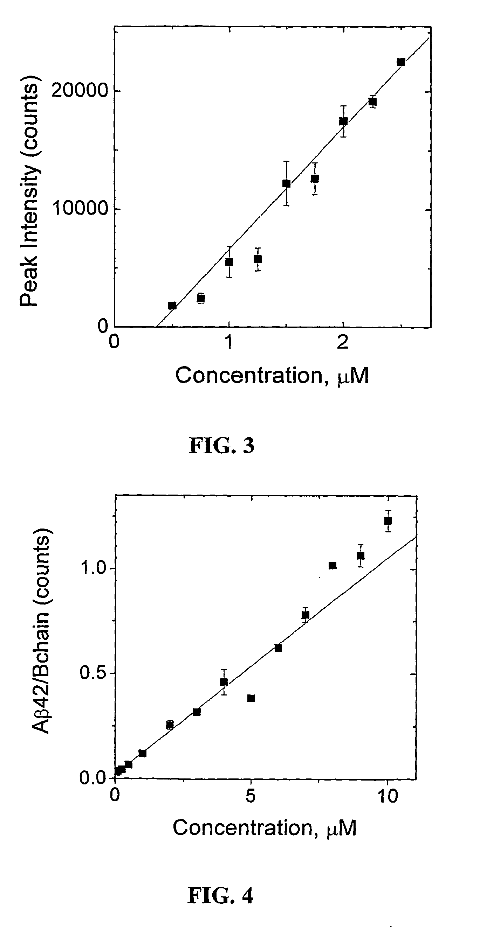

Figure 2 demonstrates that the calibration curve for B chain in a broad range of concentrations represents a saturation curve and the linear dynamic range is positioned at concentrations below 3 µM. To show the linearity in this area, we generated another calibration curve using lower B chain concentrations (Fig.3.). Data on Figure 3 confirm the quantitative nature of MALDI-TOF MS method for determination of B chain at low micromolar concentrations.

In addition to insulin B chain we also created a calibration curve for Aβ42 in the presence of 0.25 µM B chain (in the spot) by plotting the ratio of MS peak intensities against the concentration of Aβ42. (Fig.4.).

Obtained linear dependence indicates that elaborated MALDI-TOF MS method is suitable for monitoring the concentration of Aβ42 monomer, which is necessary for monitoring the aggregation of Aβ42 and also for developing the high-throughput screening test for inhibitors of this process.

### Aggregation of Aβ42 monitored by MALDI-TOF MS

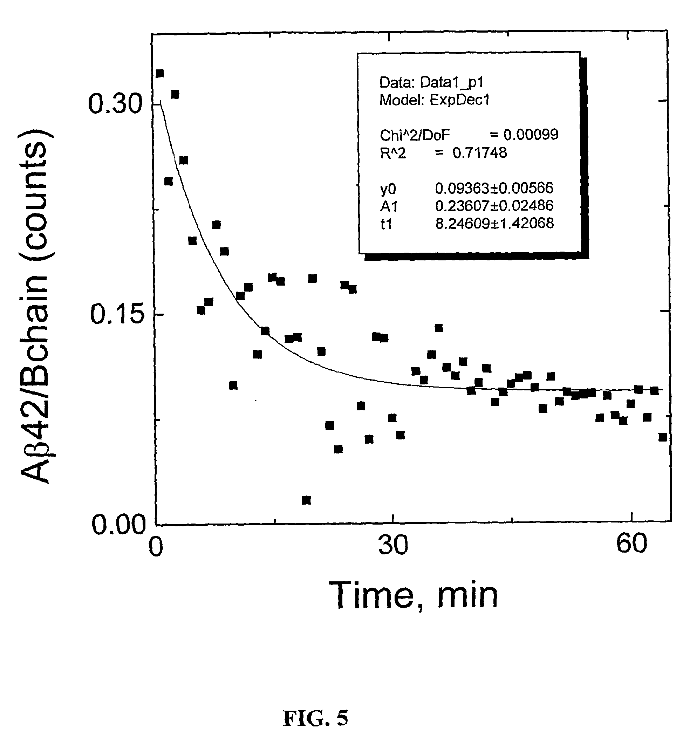

The aggregation of Aβ42 was started by diluting the alkaline stock solution into buffer with a physiological pH and aliquots at different time points were analyzed by MALDI-TOF MS. We observed a decrease in the concentration of monomeric Aβ42, which is caused by peptide aggregation. Aβ42 aggregation curve, obtained with the automatic MALDI-TOF MS method, indicates that the process is relatively fast and is completed in 15 to 20 min (Fig.5).

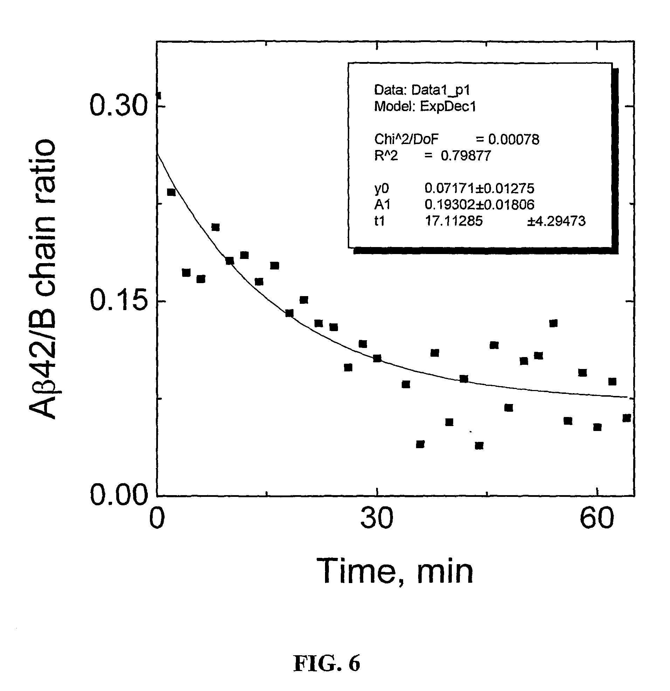

It is important to note that this result was reproducible when the same experiment was repeated on another day starting from another Aβ42 stock solution (Fig.6).

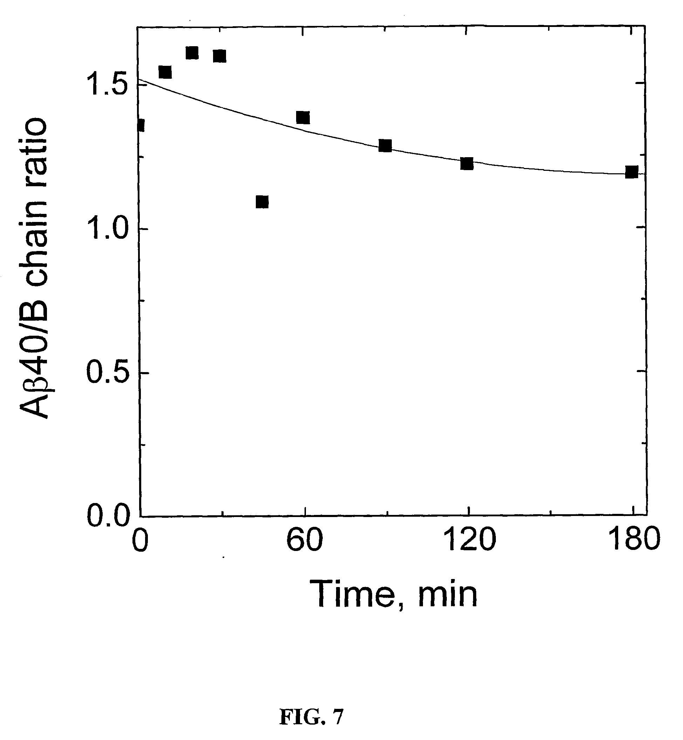

We also monitored the aggregation of Aβ40 using the same conditions and methods as in case of Aβ42. The results are presented in Figure 7.

As expected, we found that aggregation of Aβ40 is slower than that of Aβ42. Difference in the aggregation kinetics of these two peptides has also been detected in numerous previous studies (Chen and Glabe, 2006). The results of our aggregation studies indicated that Aβ42 is more suitable for developing a fast screening test for inhibitors of Aβ aggregation.

### Validation and comparison of MALDI-TOF MS assay with ThT assay

To confirm that the MALDI-TOF MS results reflect fibrillization of Aβ42, the aggregation process of Aβ42 was monitored also with the ThT assay, which is a common and recognized method for detection of amyloid fibrils.

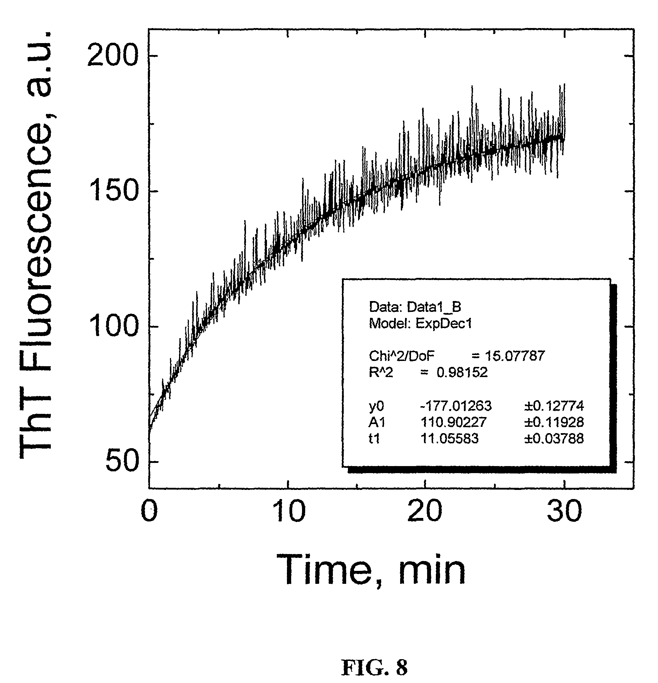

ThT assay showed similar aggregation kinetics (Fig. 8.) as observed by MALDI-TOF MS, which confirms that both assays monitor the fibrillization of Aβ42 peptide. However, these methods are different, as MALDI-TOF MS assay measures the decrease of monomeric Aβ42 peptide but ThT assay measures the formation of Aβ42 fibrils.

By comparing amounts of Aβ42 necessary for performing ThT and MALDI-TOF MS assays, we found out that the latter consumes approximately 100 times less peptide for individual measurements, which makes it more cost-effective.

### High-throughput screening of inhibitors of Aβ aggregation by MALDI-TOF MS

Elaborated automatic MALDI spotting from 96-well plates to 100-well MALDI gold plates combined with automated MS measurements enables testing of 96 samples in approximately 2.5 hours, which could be qualified as a high-throughput method. Using the elaborated assay we performed several high-throughput screening tests with potential inhibitors of Aβ42 aggregation.

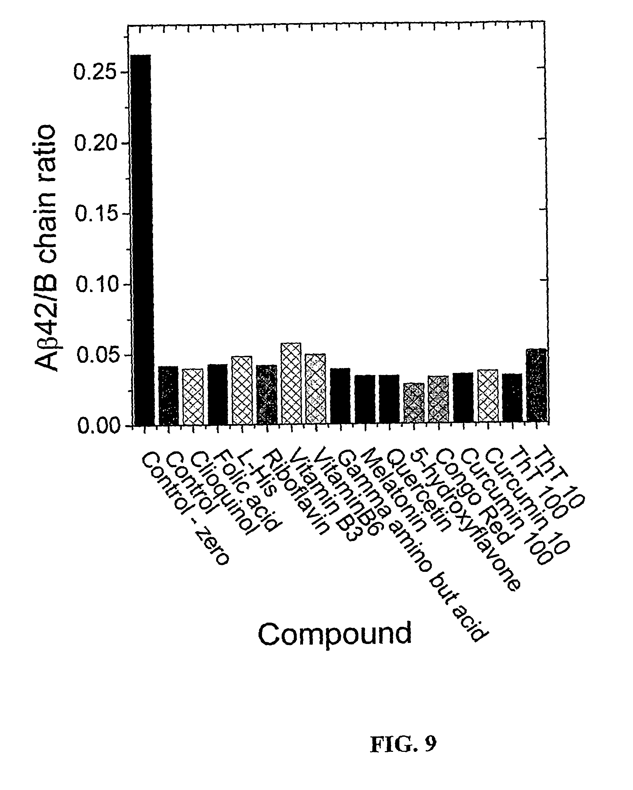

First, we tested several vitamins and some other compounds, which are known to have an inhibitory effect on Aβ42 aggregation (Fig.9.).

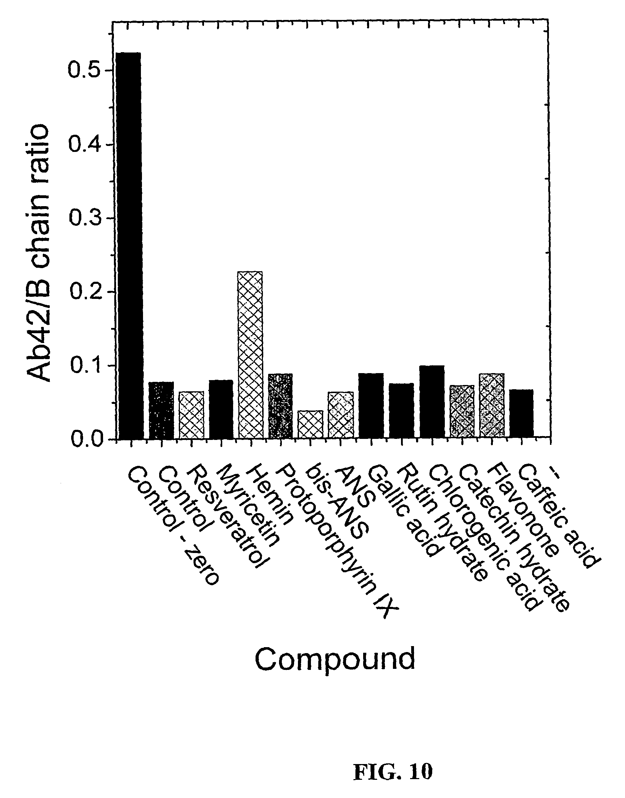

Secondly, we tested some antioxidants and other compounds, which have previously been demonstrated to exhibit inhibitory effect on Aβ42 aggregation (Fig.10).

Interestingly, 10 µM or higher concentration of almost all tested compounds did not exhibit inhibitory effect on Aβ42 aggregation in the automatic MALDI-TOF MS assay. For example, some previously identified effective inhibitors of Aβ42 aggregation, like Congo Red, Curcumin, Thioflavin T, Melatonin and Myricetin, did not exhibit inhibitory effect. Moreover, preliminary kinetic studies indicated that some compounds even accelerated the aggregation of Aβ42 instead of inhibiting the process. Reasons for discrepancy of results obtained by MALDI-TOF MS and other assays are not clear yet. However, observed differences might be connected with fundamental differences of assays that have been used in testing. We have to mention here that MALDI-TOF MS assay uses unmodified Aβ42 peptide in controlled solution conditions and is free from many disturbing factors occurring in other assays discussed previously.

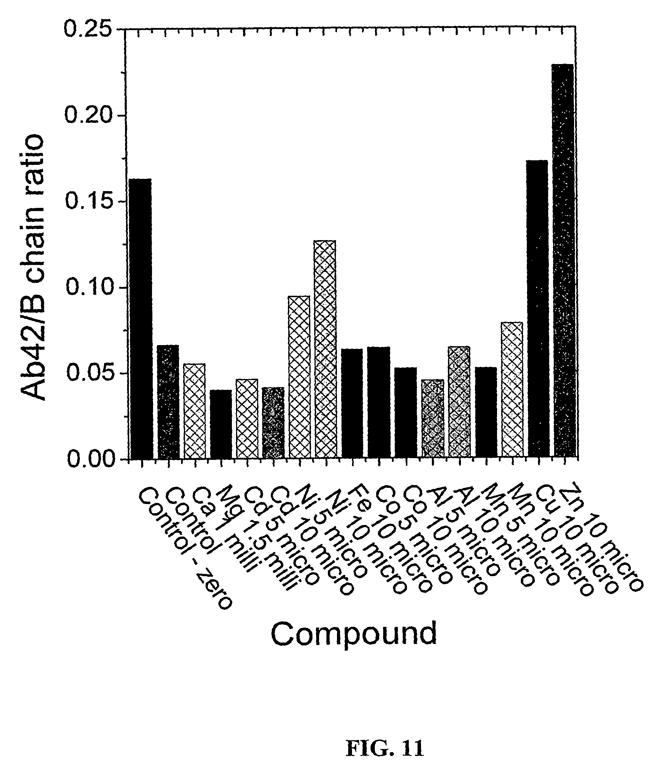

In addition to selected vitamins, antioxidants and other previously described substances, we also tested the effect of various metal ions on the aggregation of Aβ42 (Fig.11).

Figure 11 clearly shows that some metal ions exhibited a strong inhibitory effect on Aβ42 aggregation in the high-throughput screening assay.

Although this invention is described with respect to a set of aspects and embodiments, modifications thereto will be apparent to those skilled in the art. The foregoing description of the embodiments of the invention has been presented for the purposes of illustration and description. It is not intended to be exhaustive or to limit the invention to the precise form disclosed. Many modifications and variations are possible in light of this disclosure. It is intended that the scope of the invention be limited not by this detailed description, but rather by the claims appended hereto.

### REFERENCES

Beher, D., and Graham, S. L. (2005). Protease inhibitors as potential disease-modifying therapeutics for Alzheimer's disease. Expert Opin Investig Drugs 14, 1385-1409.
Blanchard, B. J., Chen, A., Rozeboom, L. M., Stafford, K. A., Weigele, P., and Ingram, V. M. (2004). Efficient reversal of Alzheimer's disease fibril formation and elimination of neurotoxicity by a small molecule. Proc Natl Acad Sci USA 101, 14326-14332.
Blennow, K., de Leon, M. J., and Zetterberg, H. (2006). Alzheimer's disease. Lancet 368, 387-403.
Chen, Y. R., and Glabe, C. G. (2006). Distinct early folding and aggregation properties of Alzheimer amyloid-beta peptides Abeta40 and Abeta42: stable trimer or tetramer formation by Abeta42. J Biol Chem 281, 24414-24422.
Cheng, X., and van Breemen, R. B. (2005). Mass spectrometry-based screening for inhibitors of beta-amyloid protein aggregation. Anal Chem 77, 7012-7015.
Esler, W. P., Stimson, E. R., Ghilardi, J. R., Felix, A. M., Lu, Y. A., Vinters, H. V., Mantyh, P. W., and Maggio, J. E. (1997). A beta deposition inhibitor screen using synthetic amyloid. Nat Biotechnol 15, 258-263.
Gelinas, D. S., DaSilva, K., Fenili, D., St George-Hyslop, P., and McLaurin, J. (2004). Immunotherapy for Alzheimer's disease. Proc Natl Acad Sci USA 101 Suppl 2, 14657-14662.
Gravina, S. A., Ho, L., Eckman, C. B., Long, K. E., Otvos, L., Jr., Younkin, L. H., Suzuki, N., and Younkin, S. G. (1995). Amyloid beta protein (A beta) in Alzheimer's disease brain. Biochemical and immunocytochemical analysis with antibodies specific for forms ending at A beta 40 or A beta 42(43). J Biol Chem 270, 7013-7016.
Haass, C., and Selkoe, D. J. (2007). Soluble protein oligomers in neurodegeneration: lessons from the Alzheimer's amyloid beta-peptide. Nat Rev Mol Cell Biol 8, 101-112.
Hardy, J. (2006a). Alzheimer's disease: the amyloid cascade hypothesis: an update and reappraisal. J Alzheimers Dis 9, 151-153.
Hardy, J. (2006b). A hundred years of Alzheimer's disease research. Neuron 52, 3-13.
Hong, H. S., Maezawa, I., Yao, N., Xu, B., Diaz-Avalos, R., Rana, S., Hua, D. H., Cheng, R. H., Lam, K. S., and Jin, L. W. (2007). Combining the rapid MTT formazan exocytosis assay and the MC65 protection assay led to the discovery of carbazole analogs as small molecule inhibitors of Abeta oligomer-induced cytotoxicity. Brain Res 1130, 223-234.
Kim, W., Kim, Y., Min, J., Kim, D. J., Chang, Y. T., and Hecht, M. H. (2006). A high-throughput screen for compounds that inhibit aggregation of the Alzheimer's peptide. ACS Chem Biol 1, 461-469.
LeVine, H., 3rd (2004). The Amyloid Hypothesis and the clearance and degradation of Alzheimer's beta-peptide. J Alzheimers Dis 6, 303-314.
McGeer, E. G., and McGeer, P. L. (2005). Abeta immunotherapy and other means to remove amyloid. Curr Drug Targets CNS Neurol Disord 4, 569-573.
Morgan, D. (2006). Immunotherapy for Alzheimer's disease. J Alzheimers Dis 9, 425-432.
Necula, M., Kayed, R., Milton, S., and Glabe, C. G. (2007). Small molecule inhibitors of aggregation indicate that amyloid beta oligomerization and fibrillization pathways are independent and distinct. J Biol Chem 282, 10311-10324.
Ritchie, C. W., Bush, A. I., and Masters, C. L. (2004). Metal-protein attenuating compounds and Alzheimer's disease. Expert Opin Investig Drugs 13, 1585-1592.
Rochet, J. C. (2007). Novel therapeutic strategies for the treatment of protein-misfolding diseases. Expert Rev Mol Med 9, 1-34.
Roher, A. E., Lowenson, J. D., Clarke, S., Woods, A. S., Cotter, R. J., Gowing, E., and Ball, M. J. (1993). beta-Amyloid-(1-42) is a major component of cerebrovascular amyloid deposits: implications for the pathology of Alzheimer disease. Proc Natl Acad Sci USA 90, 10836-10840.
Rohner, T. C., Staab, D., and Stoeckli, M. (2005). MALDI mass spectrometric imaging of biological tissue sections. Mech Ageing Dev 126, 177-185.
Selkoe, D. J. (2005). Defining molecular targets to prevent Alzheimer disease. Arch Neurol 62, 192-195.
Tanzi, R. E., and Bertram, L. (2005). Twenty years of the Alzheimer's disease amyloid hypothesis: a genetic perspective. Cell 120, 545-555.

## Claims

1. A method for screening a plurality of test compounds for inhibitors of amyloid beta (Aβ) peptide aggregation with Matrix-Assisted Laser Desorption Ionization Mass Spectroscopy (MALDI MS), comprising:
providing a matrix for MALDI MS;
providing an internal MS standard for MALDI MS, the internal MS standard comprising a solution of a known component with a suitable molecule size for quantification of MALDI MS process;
providing a monomer amyloid beta (Aβ) peptide sample in a first well of a well plate;
providing a plurality of solutions in a plurality of remaining wells of said well plate, each of said solutions comprising one test compound to be screened and said monomer Aβ peptide sample;
incubating all of the samples for a predetermined time;
mixing of said samples from said well plate with said matrix and said internal MS standard;
registering a MALDI TOF MS spectrum for said Aβ peptide and said internal MS standard;
determining a MS peak intensity from said MS spectrum;
calculating a ratio of peak intensities for said Aβ peptide and said internal MS standard; and
determining each test compound as potential therapeutic agent if the ratio of peak intensities for said monomer Aβ peptide and said internal MS standard in the presence of test compound is higher than said ratio of peak intensities for said monomer Aβ peptide and said internal MS standardalone.

2. The method of claim 1, wherein a human insulin compound is used as said internal MSstandard.

3. The method of claim 2, wherein a B chain of human insulin is used as said internal MSstandard.

4. The method of claims 1 to 3, wherein α -cyano-4-hydroxycinnamic acid is usedas saidmatrix.

5. The method of claims 1 to 4, wherein said Aβ sample is Aβ42 and said predetermined time is from about 1 to about 3 hours.

6. The method of claims I to 5, wherein said Aβ sample is Aβ40, a mixture of Aβ40 and A42, or N-terminally His-tagged Aβ42 and said predetermined time is from about 20 to 24 hours.

7. The method of claims 1 to 6, wherein said Aβ sample and said plurality of mixed test compounds are incubated in a standard 96 well plates, and said plurality of mixed test compounds are spotted to a100 well MALDI plate by using a spotting robot.

8. A method for screening a test component for Alzheimer's drug candidates, the method comprising the steps of:
preparing a first sample,comprisinga monomer Aβ peptide;
preparing a second sample, comprising said monomer Aβ peptide and a test component;
incubating said prepared samples for a predetermined time;
mixing said incubated samples with a MALDI MS matrix, to form first mixture and second mixture, wherein the MALDI-MS matrix containing a standard component;
registering a first spectrum of said first mixture by a MALDI MS, said first spectrum containing a first peak intensity for said peptide component and a second peak intensity for saidstandard component,
calculating a first ratio of said first peak intensity to said second peak intensity;
registering a second spectrum of said second mixture by the MALDI MS, said spectrum containing a third peak intensity for said peptide component and a fourth peak intensity for said standard component;
calculating a second ratio of said third peak intensity to said fourth peak intensity; and
determining said test component as a potential therapeutic agent for treating Alzheimer if said second ratio is higherthan said first ratio.

9. The method of claim 8, wherein a human insulin compound is used as said standard component.

10. The method of claim 9, wherein a B chain of human insulin compound is used as saidstandardcomponent.

11. The method of claim 8, wherein α -cyano-4-hydroxycinnamic acid is used as a matrix for the MALDI MS.

12. The method of claim 8, wherein said first sample mixture and said second sample mixture comprise Aβ42 and said predetermined time is from about I to about 3 hours.

13. The method of claim 8, wherein said Aβ sample is Aβ40, a mixture of Aβ40 and Aβ42, or N-terminally His-tagged Aβ42 and said predeterminedtimeisfromabout 20 to 24 hours.

## Patentansprüche

1. Eine Methode zum Screening einer Vielzahl von Testpräparaten für Inhibitoren von Amyloid Beta-(Aβ)-Peptid-Aggregationen mit Matrix-unterstützer Laser-Desorptions-Ionisierungsmassen-Spektroskopie (MALDI MS), bestehend aus:
Bereitstellung einer Matrix für MALDI MS;
Bereitstellung eines internen MS-Standards für MALDI MS; der interne MS-Standard beinhaltet die Lösung einer bekannten Komponente mit einer passenden Molekulgröße für die Quantifizierung des MALDI MS-Prozesses;
Bereitstellung eines Monomer-Amyloid-Beta-(Aß)-Peptidmuster in einem ersten Näpfchen einer Mikrotiterplatte;
Bereitstellung einer Vielzahl an Lösungen innerhalb einer Vielzahl von verbleibenden Näpfchen der besagten Mikrotiterplatte; jede der besagten Lösungen mit einer Testverbindung zum Screening und das besagte Monomer-Aβ-Peptidmuster;
Inkubation aller Muster für eine festgelegte Zeit;
Vermengung der besagten Muster der besagten Mikrotiterplatte mit der besagten Matrix und dem besagten internen MS-Standard;
Registrierung eines MALDI TOF MS-Spektrums für die besagten Aß-Peptide und den besagten internen MS-Standard;
Festlegung einer MS-Maximuim-Intensität des besagten MS-Spektrums;
Kalkulation eines Verhältnisses der Maximum-Intensität für die besagten Aß-Peptide und den besagten internen MS-Standard; und eine Festlegung für jedes Testverhältnis als potentieller therapeutischer Wirkstoff, falls das Verhältnis der Maximum-Intensität für das besagte Monomer Aß-Peptid und des besagten internen MS-Standard in der Gegenwart von der Testverbindung höher ist als das besagte Verhältnis der Maximum-Intensität des besagten Monomer Aß-Peptids und des besagten internen MS-Standard alleine.

2. Die Methode des Anspruchs 1, wobei eine menschliche Insulinverbindung als der besagte MS-Standard verwendet wird.

3. Die Methode des Anspruchs 2, wobei eine B-Kette menschlichen Insulins als der besagte MS-Standard verwendet wird.

4. Die Methode der Ansprüche 1 bis 3, wobei ein α-Cyano-4-Hydroxyzimtsäurederivat als die besagte Matrix verwendet wird.

5. Die Methode der Ansprüche 1 bis 4, wobei das besagte Aß-Muster Aβ42 ist und die besagte, festgelegte Zeit von etwa 1 bis zu 3 Stunden beträgt.

6. Die Methode der Ansprüche 1 bis 5, wobei das besagte Aß-Muster Aβ40, eine Mischung aus Aβ40 und Aβ42 oder N-terminales, Ilis-markiertes Aβ42 ist und die besagte, festgelegte Zeit circa 20 bis 24 Stunden beträgt.

7. Die Methode der Ansprüche 1 bis 6, wobei das besagte Aß-Muster und die besagte Vielfalt der vermischten Testverbindungen in einer Standard-96-Mikrotiterplatte inkubiert werden, und die besagte Vielfalt der gemischten Testverbindungen fleckförmig und mithilfe eines Spotting-Roboters auf einer MALDI-Platte angebracht werden.

8. Eine Screening-Methode für Testverbindungen von Alzheimer-Axzneimittelkandidaten, bestehend aus den folgenden Schritten:
Vorbereitung eines ersten Musters, einschließlich eines Monomer-Aβ-Peptids;
Vorbereitung eines zweiten Musters, einschließlich des besagten Monomer Aß-Peptids und einer Testverbindung;
Inkubation der besagten, vorbereiteten Muster für die festgelegte Zeit;
Mischen der besagten, inkubierten Muster mit einer MALDI-MS-Matrix, um die erste und die zweite Mischung zu erstellen, wobei die MALDI MS-Matrix eine Standardkomponente enthält;
Registrierung eines ersten Spektrums der besagten Mischung mit einem MALDI MS, wobei dieses erste Spektrum eine erste Maximum-Intensität für die besagte Peptid-Kompanente beinhaltet und eine zweite Maximum-Intensität für die besagte Standardkomponente;
Kalkulation eines ersten Verhältnisses der besagten Maximum-Intensität zur zweiten Maximum-Intensität;
Registrierung eines zweiten Spektrums der besagten zweiten Mischung mithilfe von MALDI MS, wobei das besagte Spektrum eine dritte Maximum-Intensität für die besagte PeptidKomponente und eine vierte Maximum-Intensität für die besagte Standardkomponente beinhaltet;
Kalkulation eines zweiten Verhälmisses der besagten dritten Maximum-Intensität zur besagten vierten Maximum-Intensität; und Bestimmung der besagten Testkomponente als ein potentieller therapeutischer Wirkstoff zur βehandlung von Alzheimer, falls das besagte zweite Verhältnis höher ist als das besagte erste Verhältnis.

9. Die Methode des Anspruchs 8, wobei ein menschliches Insulinpräparat als besagte Standardkomponente benutzt wird.

10. Die Methode von Anspruch 9, wobei eine B-Kette einer menschlichen Insulinkomponente als die besagte Standatdkomponente benutzt wird.

11. Die Methode von Anspruch 8, wobei ein α-Cyano-4-Hydroxyzimtsäurederivat als Matrix für MALDI MS benutzt wird.

12. Die Methode von Anspruch 8, wobei die besagte erste Mustermischung und die besagte zweite Mustermischung Aβ42 enthalten und die besagte und festgelegte Zeit circa 1 bis circa 3 Stunden beträgt.

13. Die Methode von Anspruch 8, wobei das besagte Aβ-Muster Aβ40, eine Mischung von Aβ40 und Aβ42, oder N-terminales, His-markiertes Aβ42 ist und die besagte festgelegte Zeit circa 20 bis 24 Stunden beträgt.

## Revendications

1. Une méthode pour cribler une diversité de molécules d'essai pour inhibiteurs de l'agrégation de peptides de Bêta-amyloïde [*Aβ*]) par Spectrométrie de Masse à Désorption-Ionisation Laser Assistée par Matrice *(MALDI MS*)*,* comprenant :
une matrice pour la *MALDI MS ;*
un étalon interne de MS pour la *MALDI MS,* l'étalon interne de MS comprenant une solution d'une molécule connue avec un poids moléculaire adapté pour le processus de quantification de *la MALDI MS ;*
un échantillon d'un monomère de peptide de Bêta-amyloïde (*Aβ*) dans le premier puits d'une plaque à puits ;
une multitude de solutions dans la multitude des puits restants de ladite plaque, chacune desdites solutions comprenant une molécule d'essai à être criblée et ledit échantillon d'un monomère de peptide d'*Aβ* ;
l'incubation de l'ensemble des échantillons pour un temps prédéterminé ;
le mélange desdits échantillons de ladite plaque avec ladite matrice et ledit étalon interne de *MS ;*
l'enregistrement du spectre de la *MALDI TOF MS* pour ledit peptide d'*Aβ* et ledit étalon interne de *MS* ;
la détermination d'un pic d'intensité de *MS* dudit spectre de *MS* ;
le calcul du rapport des intensités de pics pour ledit peptide d'*Aβ* et ledit étalon interne de *MS* ; et
la détermination de chaque molécule d'essai comme un agent thérapeutique potentiel si le rapport des intensités de pics pour ledit monomère de peptide d'*Aβ* et ledit étalon interne de *MS* en présence d'une molécule d'essai est supérieur au dit ratio des intensités de pics pour ledit monomère de peptide d'*Aβ* et ledit étalon interne de *MS* tout seuls.

2. La méthode de la revendication 1, où une molécule d'insuline humaine est utilisée comme ledit étalon interne de *MS.*

3. La méthode de la revendication 2, où une chaîne B d'insuline humaine est utilisée comme ledit étalon interne de *MS.*

4. La méthode des revendications 1 à 3, où l'acide a -cyano-4-hydroxycinnamique est utilisé comme ladite matrice.

5. La méthode des revendications 1 à 4, où ledit échantillon *Aβ* est *Aβ42* et ledit temps prédéterminé est d'environ 1 heure à environ 3 heures.

6. La méthode des revendications 1 à 5, où ledit échantillon *Aβ* est *Aβ40,* un mélange *d'Aβ40* et *d'Aβ42,* ou *Aβ42* marqué à l'extrémité N-terminale d'une étiquette-His et ledit temps prédéterminé est d'environ 20 à 24 heures.

7. La méthode des revendications 1 à 6, où ledit échantillon *Aβ* et ladite multitude de molécules d'essai mélangées sont incubés dans une plaque 96 puits normale, et ladite multitude de molécules d'essai mélangées sont spottées sur une plaque *MALDI* 100 puits en utilisant un robot spotter.

8. Une méthode pour cribler une molécule d'essai pour des candidats comme médicament pour la maladie d'Alzheimer, la méthode comprenant les étapes de :
préparation d'un premier échantillon, comprenant un monomère de peptide d'*Aβ* ;
préparation d'un second échantillon, comprenant un monomère de peptide d'*Aβ* et une molécule d'essai ;
incubation desdits échantillons préparés pour un temps prédéterminé mélange desdits échantillons incubés avec une matrice de *MALDI MS,* Pour former un premier mélange et un second mélange, où la matrice de *MALDI-MS* contient un composant étalon ;
enregistrement d'un premier spectre dudit premier mélange par *MALDI MS,* ledit premier spectre contenant une première intensité de pic pour ledit composant peptide et une seconde intensité de pic pour ledit composant étalon ;
calcul d'un premier rapport de ladite première intensité de pic à ladite seconde intensité de pic ;
enregistrement d'un second spectre dudit second mélange par *MALDI MS,* ledit second spectre contenant une troisième intensité de pic pour le composant peptide et une quatrième intensité de pic pour ledit composant étalon;
calcul d'un second rapport de ladite troisième intensité de pic à ladite quatrième intensité de pic; et
détermination de ladite molécule d'essai comme un agent thérapeutique potentiel pour le traitement de la maladie d'Alzheimer si ledit second rapport est supérieur au dit premier rapport.

9. La méthode de la revendication 8, où une molécule d'insuline humaine est ledit composant étalon.

10. La méthode de la revendication 9, où une chaîne B de molécule d'insuline humaine est utilisée comme ledit composant étalon.

11. La méthode de la revendication 8, où l'acide α -cyano-4-hydroxycinnamique est utilisé comme une matrice pour la *MALDI MS.*

12. La méthode de la revendication 8, où ledit premier mélange d'échantillon et ledit second mélange d'échantillon comprennent *Aβ42* et ledit temps prédéterminé est d'environ 1 à environ 3 heures.

13. La méthode de la revendication 8, où ledit échantillon *Aβ* est *Aβ40,* un mélange d*'Aβ40* et *Aβ42,* ou *Aβ42* marqué à l'extrémité N-terminale d'une étiquette-His et ledit temps prédéterminé est d'environ 20 à 24 heures.
